Europäisches Patentamt

European Patent Office

Office européen des brevets

(19)

(11) **EP 0 695 805 B1**

(12) **EUROPÄISCHE PATENTSCHRIFT**

(45) Veröffentlichungstag und Bekanntmachung des Hinweises auf die Patenterteilung:
**19.05.2004   Patentblatt 2004/21**

(51) Int Cl.⁷: **C12Q 1/32**, G01N 33/72

(21) Anmeldenummer: **95112145.8**

(22) Anmeldetag: **02.08.1995**

(54) **Verfahren zur Analyse einer medizinischen Probe unter Vermeidung von Störbeiträgen aufgrund von Hämolyse**

Method for analysing a medical sample, preventing disturbancies caused by hemolysis

Méthode d'analyse d'un échantillon médical avec prévention de dérangements causés par l'hémolyse

(84) Benannte Vertragsstaaten:
**CH DE ES FR GB IT LI**

(30) Priorität: **03.08.1994   DE 4427492**

(43) Veröffentlichungstag der Anmeldung:
**07.02.1996   Patentblatt 1996/06**

(73) Patentinhaber: **Roche Diagnostics GmbH**
**68305 Mannheim (DE)**

(72) Erfinder:
 • **Wild, Thomas, Dr.**
   **D-82362 Weilheim (DE)**
 • **Weber, Friederike**
   **D-80803 München (DE)**
 • **Berding, Christoph, Dr.**
   **D-81667 München (DE)**
 • **Kleider, Wilhelm**
   **D-82418 Murnau (DE)**

(74) Vertreter: **Böhm, Brigitte, Dipl.-Chem. Dr. et al**
**Weickmann & Weickmann**
**Patentanwälte**
**Kopernikusstrasse 9**
**81679 Munich (DE)**

(56) Entgegenhaltungen:
   **EP-A- 0 139 985        EP-A- 0 268 025**
   **DE-A- 2 847 176        GB-A- 2 026 692**

 • **PATENT ABSTRACTS OF JAPAN vol. 012 no. 368 (P-766) ,4.Oktober 1988 & JP-A-63 120243 (HITACHI LTD) 24.Mai 1988,**

**Beschreibung**

Das vorliegende Patent betrifft ein Verfahren zur Analyse einer medizinischen Probe unter Vermeidung von Meßfehlern aufgrund von Hämolyse.

Das häufigste Untersuchungsmaterial für biochemische Analysen ist Blutserum oder Plasma. Es ist bekannt, daß bei der Bestimmung verschiedener Analyten aus Blutserum oder Blutplasma Fehler auftreten können, die das Ergebnis der Messung beeinflussen und die durch die Eigenschaften der Probe verursacht werden. Meßfehler in diesem Sinne kommen insbesondere durch hämolytisches Probengut zustande. Die Hämolyse kann bei Blutproben in besonderem Maße die analytischen Meßergebnisse verfälschen.

Wenn bei einer Blutprobe Hämolyse auftritt, dann ist ein Teil der roten Blutkörperchen zerstört worden, und die dabei freiwerdenden Inhaltsstoffe kontaminieren das Probengut. Dies hat zur Folge, daß durch die Inhaltsstoffe der Blutkörperchen, wie z.B. Hämoglobin, die analytischen Serum- oder Plasmawerte verfälscht werden.

Zur Behebung dieses Nachteils wird in der Patentschrift US-A-4,263,512 die Bestimmung von interferierendem Chromogen in einer Blutprobe zusammen mit dem Analyten vorgeschlagen und die Korrektur des Meßfehlers gemäß dem Grad der Hämolyse (korreliert mit Chromogenkonzentration) empfohlen. Bei dieser konventionellen Korrekturmethode wird jedoch nur der von Erythrozyten freigesetzte rote Blutfarbstoff für die Bestimmung des Meßfehlers bei hämolytischem Probenmaterial herangezogen. In der EP-0 268 025 B1 wird darauf hingewiesen, daß ein quantitativer Zusammenhang zwischen dem Grad der Hämolysestörung, der Analytkonzentration und dem durch die Störung erzeugten Meßfehler besteht. Dieser Zusammenhang läßt sich mit Hilfe der multiplen Regression darstellen. Die aus dieser Anpassung ableitbaren Korrekturfaktoren erlauben dann auf der Basis einer unabhängig bestimmten Hämolysestörung, z.B. über eine Hb-Wertmessung (unter Vernachlässigung eventueller zusätzlicher Abhängigkeiten von Analytkonzentration) die Berichtigung des Analysenresultats.

Der klinisch chemische Analyzer "Synchron CX 5" der Firma Beckman benutzt ein Set von 2 bis 5 Wellenlängen für die Messung von Reaktionsverläufen und Kompensation von störendem Background. Über eine geeignete Auswahl nicht reaktionsrelevanter zusätzlicher Wellenlängen ist es möglich, endogene spektrale Interferenzen automatisch zu korrigieren. Im 16-Sekundentakt erfolgt ein "eight flash photometric measurement", d.h. bei max. 5 Wellenlängen werden 8 x 5 = 40 Meßdaten generiert. Für die Signalkorrektur wird dann die folgende polychromatische Gleichung verwendet:

$$\text{Absorptionsdifferenz} = \text{Differenz} (A - B - C - D - E + k)$$

wobei

A =   Absorptionsänderung der bichromatisch gemessenen Analytreaktion
B - E =   gewichtete bichromatische Korrektur-Wellenlängen und
k =   Konstante, mit der der spektrale Einfluß von Seren in Abwesenheit von Lipämie, Hämolyse oder Ikterus berücksichtigt wird.

Abhängig vom Analysenautomat kann die polychromatische Korrektur, wenn sie Bestandteil einer Testapplikation ist, entweder kategorisch (Beckman-Analyzer) erfolgen, oder aber erst oberhalb eines testspezifischen Grenzwertes, ab welchem der Interferenzfehler die Probenwiederfindung sichtbar beeinflußt.

Aufgabe der vorliegenden Erfindung war es jedoch, eine Analysenmethode bereitzustellen, die es ermöglicht, Meßfehler durch kontaminierende Bestandteile in einer Blutserumprobe oder in einer Blutplasmaprobe von hämolytischem Blut mit einer gegenüber herkömmlichen Korrekturverfahren verbesserten Richtigkeit und deutlich reduziertem Arbeitsaufwand zu bestimmen.

Gelöst wird die Aufgabe durch ein Verfahren zur Analyse einer medizinischen Probe unter Vermeidung von Meßfehlern aufgrund von Hämolyse, bei dem vor der eigentlichen photometrischen Bestimmung einer in der Probe enthaltenen Komponente die Probe einer Vorreaktion unterworfen wird, durch die der Hämolysegrad der Probe bestimmt wird, und der nachfolgend erhaltene Meßwert der zu bestimmenden Komponente korrigiert wird um einen Wert, der durch Korrelation des Hämolysegrades mit dem Meßfehlerbeitrag von störenden Komponenten ermittelt wurde.

Im Vergleich zum Stand der Technik zeichnet sich das erfindungsgemäße Verfahren durch den Vorteil aus, daß für die Korrektur des Meßwertes einer hämolytischen Probe keine unabhängige Bestimmung des Hämolysegrades, z.B. durch Bestimmung des Hämoglobinwertes in einer Probe, erforderlich ist. Vielmehr konnte ein Zusammenhang zwischen dem Hämolysegrad einer Probe und der bei hämolytischen Seren oder Plasmen festgestellten Vorreaktion festgestellt werden. Die Vorreaktion läuft hierbei in Gegenwart von Probe und einem Reagenz ab, jedoch vor Zugabe des Startreagenzes der eigentlichen Meßwertbestimmung.

Mit Hilfe des in der vorliegenden Erfindung beschriebenen biometrischen Modells ist es möglich, den Hämolysegrad einer Probe direkt aus der von ihr verursachten Vorreaktion abzuschätzen und eine entsprechende Korrektur des Analysenresultats durchzuführen. Als weiterer Fehlerbeitrag für die Analyse ist zu berücksichtigen, daß bei Bestimmung bestimmter Substanzen ebenfalls ein falsches Resultat für hämolytische Proben erhalten werden kann, wenn diese Substanz auch in roten Blutkörperchen vorhanden ist, und dadurch nach Hämolyse ebenfalls zusätzlich in der zu analysierenden Probe vorhanden ist. Auch dieser Meßfehlerbeitrag wird durch das erfindungsgemäße Verfahren berücksichtigt, da in die Vorreaktion automatisch neben dem Hämolysegrad auch der Analytgehalt der Probe eingeht.

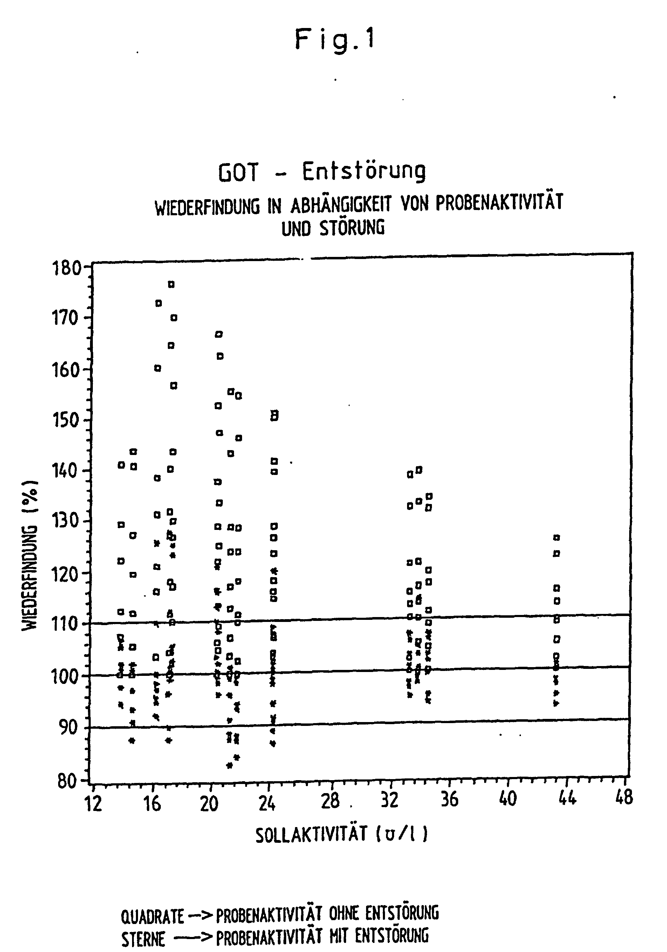

Das erfindungsgemäße Verfahren erlaubt eine Korrektur von Hämolysemeßfehlern und bewirkt im Referenzbereich (31 U/l bei Frauen, 37 U/l bei Männern) eine besonders effiziente Korrektur (< 10 % Wiederfindungserhöhung (s. Figur 1)).

Zusätzlich konnten außerhalb des Referenzbereiches Störungen bis zu einer Größenordnung von 80 %, die mit Verfahren gemäß dem Stand der Technik erhalten wurden, mit der entsprechenden Korrektur auf kleiner gleich 30 % reduziert werden.

Das erfindungsgemäße Verfahren berücksichtigt allein über die Vorreaktion die individuellen Eigenschaften der Probe, so daß basierend auf den bisherigen experimentellen Daten weitere spezifische Korrekturverfahren für das Probegut entfallen. Es sind insbesondere keine zusätzlichen Messungen erforderlich. Dies ist ein bedeutender Vorteil gegenüber den bisher beschriebenen Korrekturverfahren für hämolytische Seren oder Plasmen.

In einer bevorzugten Ausführungsform der Erfindung wird die Vorreaktion ebenfalls photometrisch erfaßt. Es ist hierbei besonders bevorzugt, daß die photometrische Bestimmung bichromatisch, und inbesondere bei 340/405 nm erfolgt und der eigentliche Meßwert durch Differenzbildung der Ergebnisse beider Wellenlängen zustande kommt.

Die Einleitung der Vorreaktion erfolgt bevorzugt durch Zugabe eines Reagenzes, welches NADH und LDH enthält. In einer besonders bevorzugten Ausführungsform enthält das Reagenz zusätzlich MDH.

In einer weiteren bevorzugten Ausführungsform der vorliegenden Erfindung wird die Beziehung zwischen dem durch die Vorreaktion bestimmten Hämolysegrad und dem Meßfehlerbeitrag durch die störenden Komponenten, wie insbesondere Hämoglobin oder auch der zu bestimmenden Komponente, die jedoch aufgrund der Hämolyse in der Probe vorhanden ist, auf einer breiten Basis für eine große Probandengruppen heterogenen Gesundheitszustandes, Alters und Geschlechts abgesichert.

In einem besonders bevorzugten Verfahren wird der Zusammenhang zwischen der Vorreaktion und der Hauptreaktion bezüglich der zu bestimmenden Komponente mit Hilfe der Formel

$$\text{Rate}_{\text{Substanz/Probe}} = \text{Rate}_{\text{total}} - \text{Rate}_{\text{Vorreaktion}} - \text{Rate}_{\text{Substanz/Erythrozyten}}$$

definiert, wobei Substanz die zu bestimmende Komponente in der Probe bedeutet.

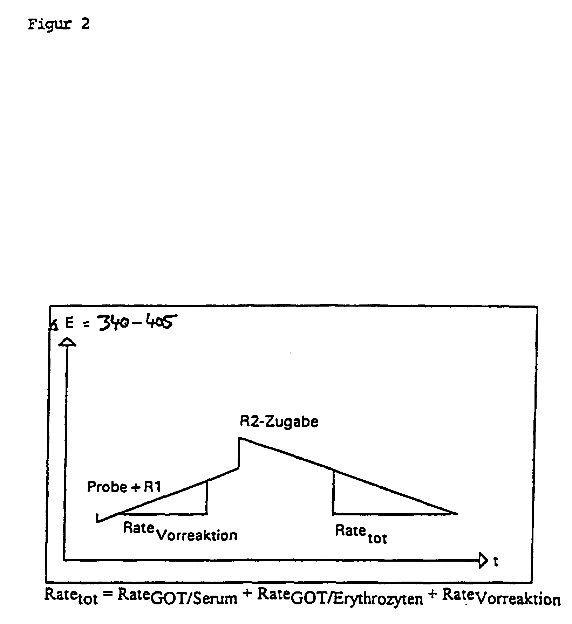

Eine Möglichkeit zur Entstörung konnte nämlich mit Hilfe eines mathematischen Zusammenhangs zwischen der Vorreaktion und der Hauptreaktion festgestellt werden. Ein schematischer Reaktionsverlauf ist in Figur 2 dargestellt und der mathematische Zusammenhang für die Gesamtreaktion lautet

$$\text{Rate}_{\text{total}} = \text{Rate}_{\text{Substanz/Probe}} + \text{Rate}_{\text{Substanz/Erythrozyten}} + \text{Rate}_{\text{Vorreaktion}},$$

wobei mit Substanz die zu bestimmende Komponente gemeint ist, die entweder in Serum oder in Erythrozyten enthalten ist. Dies bedeutet, daß sich die Meßwertänderung pro Zeiteinheit (Gesamtkinetik) aus dem Signal des hämolysefreien Serums sowie dem der Erythrozyten und der Vorreaktion, die über die Zugabe des eigentlichen Bestimmungsreagenzes hinaus andauert, zusammensetzt. Um hieraus den störungsfreien Serumwert zu berechnen, muß die Formel folgendermaßen aufgelöst werden:

$$\text{Rate}_{\text{Substanz/Probe}} = \text{Rate}_{\text{total}} - \text{Rate}_{\text{Substanz/Erythrozyten}} - \text{Rate}_{\text{Vorreaktion}}$$

und führt zu der oben genannten Formel zur Bestimmung von $\text{Rate}_{\text{Substanz/Probe}}$.

Mit dem erstellten Datensatz kann anhand einer multiplen linearen Regression folgendes Modell zur Berechnung der $\text{Rate}_{\text{Substanz/Erythrozyten}}$ herangezogen werden:

$$\text{Rate}_{\text{Substanz/Erythrozyten}} = \alpha \times \text{Rate}_{\text{Vorreaktion}} + \beta \times (\text{Rate}_{\text{Vorreaktion}})^2$$

(s. Figur 4)

Im Rahmen der vorliegenden Erfindung ist es besonders bevorzugt, daß die für die Berechnung des korrigierten Wertes erforderliche Formel auf einem Datenträger gespeichert wird und dieser für eine automatische Korrektur der Analysenergebnisse mit Hilfe der elektronischen Datenverarbeitung verwendet wird.

Besonders bevorzugt ist, wenn der Wert des Meßfehlers bereits durch die auf dem Datenträger gespeicherten Informationen berechnet werden kann. Hierzu werden dem Datenträger die Daten eingespeichert, die für die Beziehung zwischen dem durch die Vorreaktion bestimmten Hämolysegrad und dem Meßfehlerbeitrag für eine große Probandengruppe ermittelt wurde.

Besonders bevorzugt wird das Verfahren so angewandt, daß der korrigierte Wert der gestörten Komponente auf dem Ausdruck und/oder auf dem Display einer elektronischen Datenverarbeitung angezeigt wird, welche für die Messung eingesetzt wird. Es ist dabei wiederum bevorzugt, daß der korrigierte Wert mit einem Toleranzbereich angegeben wird. Jedoch kann es zweckmäßig sein, die Korrektur des Meßwertes nur innerhalb bestimmter Toleranzgrenzen automatisch durchführen zu lassen und außerhalb dieser Toleranzgrenzen Korrekturen nach entsprechender Berücksichtigung der Umstände von Hand durchzuführen.

Das erfindungsgemäße Verfahren wird besonders bevorzugt bei zu bestimmenden Komponenten angewandt, die ausgewählt sind aus (a) der Analytgruppe, die entsprechend dem biochemischen Reaktionstypus von GOT/GPT detektiert wird und/oder (b) zu einer Analytgruppe gehört, die aus Gesamtprotein, Albumin, LDH, Kalium, Gesamtcholesterin, freiem Cholesterin, Harnsäure, Triglyceriden, Natrium, Chlorid, β-Lipoproteinen, Thymoltrübungstesten, Zinksulfattrübungstesten, Phospholipiden und freien Fettsäuren besteht. Es ist dabei auch möglich und eine bevorzugte Ausführungsform der Erfindung, eine Komponente zu bestimmen, deren Konzentration in roten Blutzellen höher ist als im Blutserum oder im Blutplasma.

Das erfindungsgemäße Verfahren stellt also eine leicht automatisierbare und schnell durchführbare Möglichkeit dar, in Blut, Blutserum oder Plasma enthaltenden Proben enthaltene Komponenten zu bestimmen und hierbei Meßfehlern aufgrund von Hämolyse zu vermeiden, wobei nur zusätzlich zur der eigentlichen Bestimmungsreaktion eine Vorreaktion durchgeführt wird, welche die Bestimmung des Hämolysegrades erlaubt.

Die vorliegende Erfindung wird durch die beigefügten Figuren und die Beispiele weiter erläutert.


BEISPIEL 1

Experimente zur Bestimmung des Parameters Glutamat-Oxalacetat-Transaminase (GOT)

Die GOT-Aktivität wird durch hämolytisches Probenmaterial stark gestört und führt zu falsch positiven Ergebnissen. Eine deutliche Abhängigkeit zwischen Hämolysegrad und Erhöhung der GOT-Aktivität liegt vor. Bei Serumwerten von ca. 20 U/l wird GOT bei einem Hb-Wert von 500 mg/dl um 80 % mit Verfahren gemäß des Standes der Technik zu hoch gemessen. Diese Störung beruht vor allem darauf, daß GOT in den Erythrozyten enthalten ist und damit ein direkter Bezug zwischen Hämolysegrad und Aktivitätserhöhung besteht.


Testprinzip des GOT-Tests:

$\alpha$-Ketoglutarat + L-Aspartat $\overset{GOT}{}$ L-Glutamat + Oxalacetat   Oxalacetat + NADH + H$^+$ $\overset{MDH}{}$ L-Malat + NAD$^+$

Die Messung erfolgt bei 340 nm (Hauptwellenlänge) und 405 nm (Nebenwellenlänge). Detektiert wird der NADH-Abbau.


Hämolysatherstellung 1:

Blutabnahme (Heparin-Plasma)/Zentrifugieren/Überstand verwerfen/Blutkuchen mit 0,9 % NaCl 3x waschen und Erythrozyten mit Wasser zum Platzen bringen und über Glaswolle filtrieren/Hb-Gehalt bestimmen.
Zum Aufstocken Serum vom selben Spender verwerfen.


Hämolysatherstellung 2:

Blutabnahme(Serum)/Zentrifugieren/Serumaufbewahren/Blutkuchen mit Glasperlen versetzen und 2 - 3 Stunden kräftig rühren/Zentrifugieren (Glasperlen dienen als Trennschicht zwischen Zellrückständen und Hämolysat) /Überstand abpipettieren und Hb-Gehalt bestimmen.

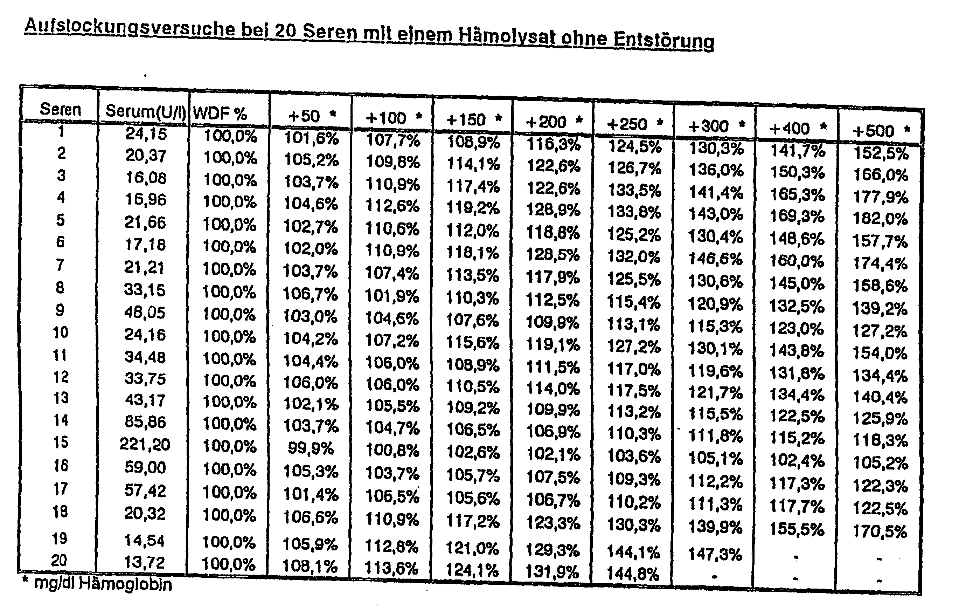

Die folgenden Resultate wurden unter Anwendung der Hämolysatherstellung 2 mit 20 verschiedenen Seren in unterschiedlichen Konzentrationsbereichen erhalten.
Die Aufstockung der o.g. Seren erfolgte in 8 Schritten: 50, 100, 150, 200, 250, 300, 400, 500 mg/dl Hb (Figur 3).

Die Messungen wurden am klinisch chemischen Analysenautpmaten Hitachi 717 mit den Meßpunkten 10 - 20 (Vorreaktion) und 30 - 50 (Hauptreaktion) durchgeführt.

Um das oben beschriebene Korrekturverfahren zu validieren, wurde zunächst ein Panel von nicht hämolytischen Humanseren am Analysenautomat Hitachi 717 vermessen und die gefundenen GOT-Aktivitäten als BM-Sollwerte definiert.

Daraufhin wurden 25 Seren aus diesem Panel mit bis zu 8 verschiedenen Hämolysat-Aufstockungen (insgesamt 217 Proben) vermessen.

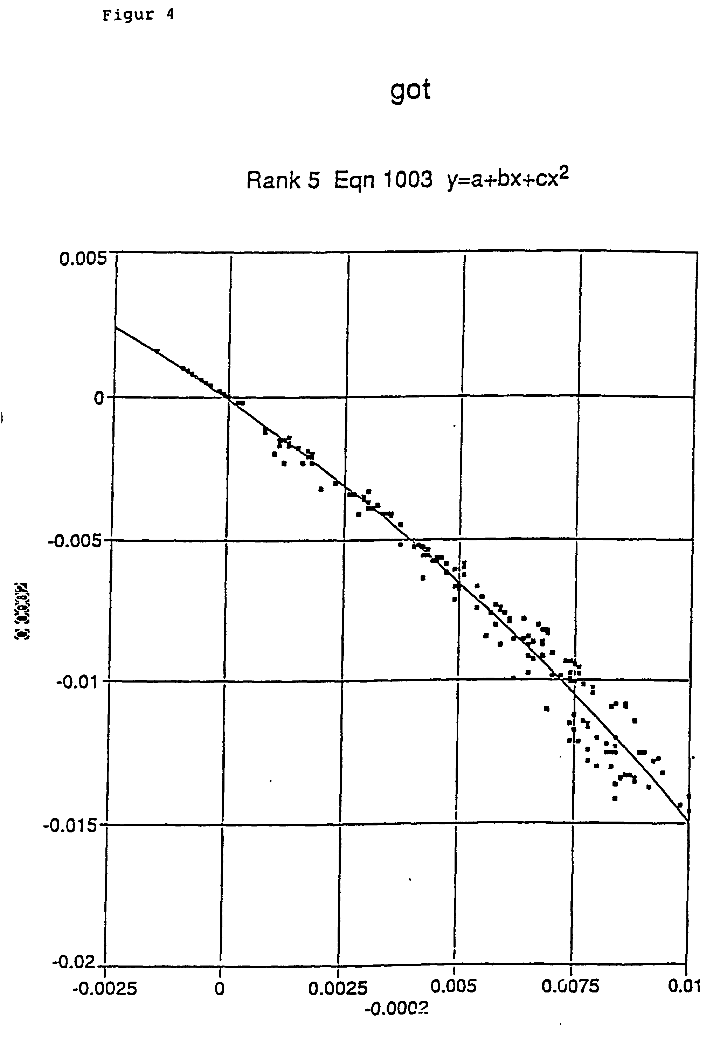

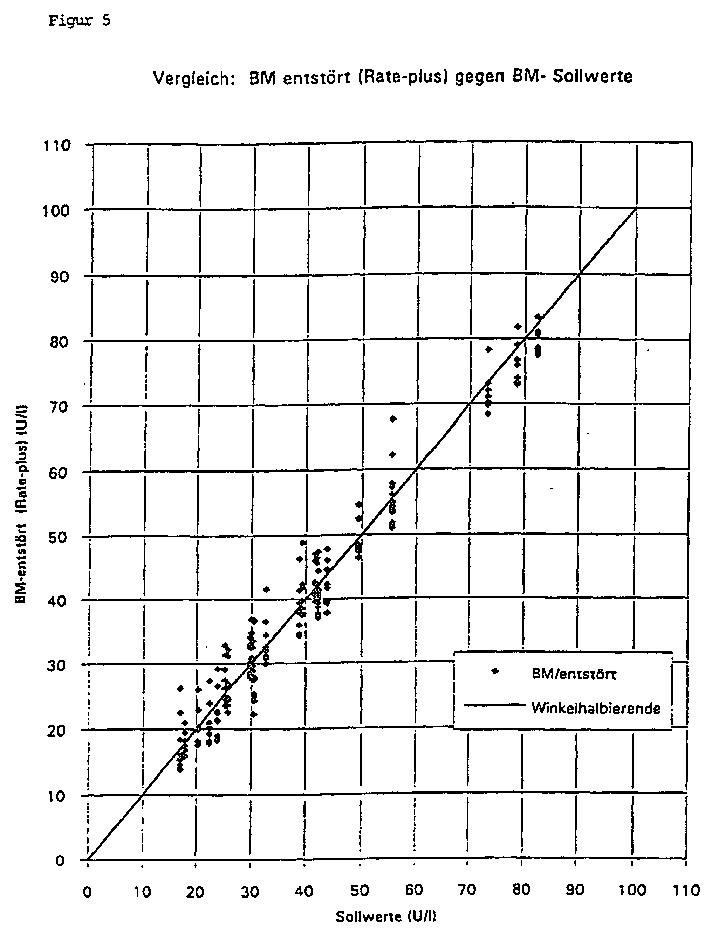

Die Methodenvergleiche zwischen den korrespondierenden Meßwerten von nicht hämolytischem ("Sollwert") und hämolytischem Probengut ("Istwert") ohne und mit Korekturverfahren zeigen die Figur 4 (Korrelation zwischen Soll- und Istwerten = 0,923) und die Figur 5 (Korrelation zwischen Soll- und Istwerten = 0,981). Dieses mehrfach reproduzierte Ergebnis zeigt deutlich, daß die hämolytische Interferenz über das hier angegebene Verfahren effizient korrigiert werden kann.

**Klärung der Vorreaktion:**

1. Einfluß des Reagenzes

| Zusammensetzung Reagenz 1: | |
| --- | --- |
| 1. Natriummonohydrogenphosphat<br>2. Natriumdihydrogenphosphat<br>3. L(+)-Mono-Natriumaspartat | Puffer A |
| 4. LDH<br>5. NADH<br>6. MDH | Enzymtabletten + Füllstoffe |

Konservierungsmittel: Na-Azid

In Austauschversuchen konnte geklärt werden, was den Anstieg der Vorreaktion bei 340/405 nm verursacht, und wie sich hierbei die Hauptreaktion verhält:

| Puffer + hämolytische Probe | Vorreaktion | Hauptreaktion |
|---|---|---|
| | | |
| Original | ⇑⇑ | ⇓⇓ |
| Puffer A | -- | -- |
| " + LDH | -- | -- |
| " + MDH | -- | -- |
| " + NADH | ⇓ | ⇓ |
| " + LDH + MDH + NADH | ⇑ | ⇓⇓ |
| " + MDH + LDH | - | - |
| " + MDH + NADH | ⇓ | ⇓⇓ |
| " + LDH + NADH | ⇑ | ⇓ |

Bei der Zugabe des Reagenzes R1 zu einer hämolytischen Probe tritt mit steigendem Hämolysegrad eine Vorreaktion mit Signalanstieg auf. Die Messung erfolgt bichromatisch bei 340/405 nm.

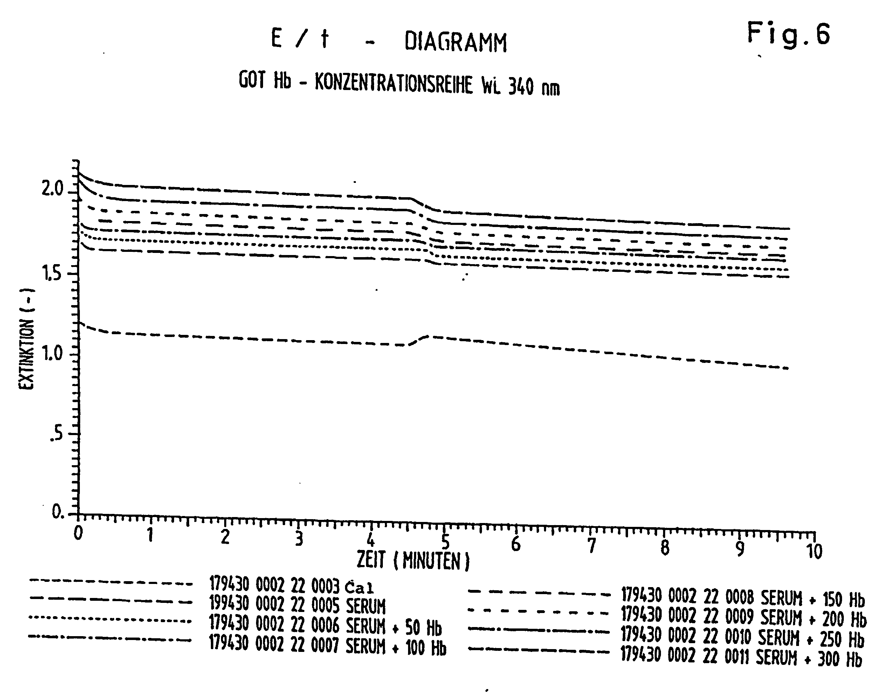

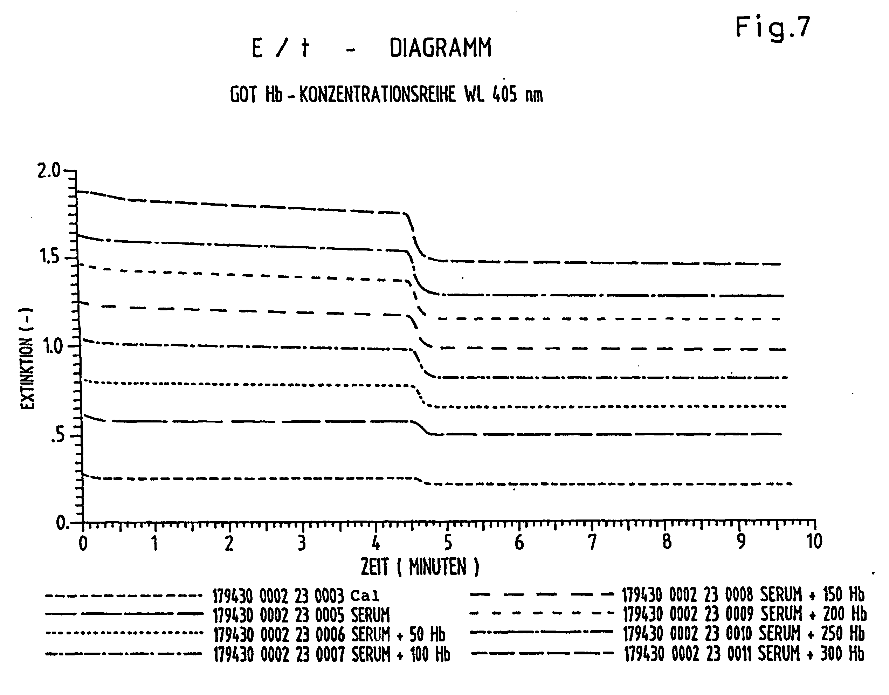

Wird dieselbe Messung bei 340 nm durchgeführt, kann keine Vorreaktion festgestellt werden. Bei 405 nm hingegen findet eine Reaktion mit abnehmendem Signal statt (Figuren 6 und 7). Die steigende Vorreaktion kommt demnach aufgrund der Differenzbildung zwischen den beiden Wellenlängen zustande.

**Patentansprüche**

1. Verfahren zur Analyse einer medizinischen Probe unter Vermeidung von Störbeiträgen aufgrund von Hämolyse, bei dem vor der Hauptreaktion für eine in der Probe enthaltene Komponente eine Vorreaktion erzeugt und gemessen wird, durch die der Hämolysegrad der Probe bestimmt wird und das nachfolgend erhaltene Ergebnis der zu bestimmenden Probe um diesen Störbeitrag unter Ausnutzung des gefundenen Zusammenhangs (Korrelation) zwischen dem Hämolysegrad und dem Störbeitrag korrigiert wird.

2. Verfahren nach Anspruch 1,
   **dadurch gekennzeichnet,**
   **daß** die Vorreaktion ebenfalls photometrisch erfaßt wird.

3. Verfahren nach Anspruch 1 oder 2,
   **dadurch gekennzeichnet,**
   **daß** die photometrische Bestimmung bichromatisch bei 340/405 nm erfolgt und der eigentliche Meßwert durch Differenzbildung der Ergebnisse beider Wellenlängen zustande kommt.

4. Verfahren nach einem der vorhergehenden Ansprüche,
   **dadurch gekennzeichnet,**
   **daß** die Einleitung der Vorreaktion durch Zugabe eines Reagenzes erfolgt, welches NADH und LDH enthält.

5. Verfahren nach Anspruch 4,
   **dadurch gekennzeichnet,**

**daß** das Reagenz zusätzlich MDH enthält.

**6.** Verfahren nach einem der vorhergehenden Ansprüche,
**dadurch gekennzeichnet,**
**daß** die Beziehung zwischen dem durch die Vorreaktion bestimmten Hämolysegrad und dem Meßfehlerbeitrag durch die störenden Komponenten auf einer breiten Basis für eine große Probandengruppe heterogenen Gesundheitszustandes, Alters und Geschlechts abgesichert ist.

**7.** Verfahren nach einem der vorhergehenden Ansprüche,
**dadurch gekennzeichnet,**
**daß** die Korrelation der Vorreaktion mit der Bestimmungsreaktion bezüglich der zu bestimmenden Komponente erfolgt über die Formel

$$Rate_{Substanz/Probe} = Rate_{total} - Rate_{Vorreaktion} - Rate_{Substanz/Erythrozyten}$$

wobei Substanz die zu bestimmende Komponente in der Probe bedeutet.

**8.** Verfahren nach einem der vorhergehenden Ansprüche,
**dadurch gekennzeichnet,**
**daß** die für die Berechnung des korrigierten Wertes erforderliche Formel auf einem Datenträger gespeichert wird und dieser für eine automatische Korrektur der Analysenergebnisse mit Hilfe der elektronischen Datenverarbeitung verwendet wird.

**9.** Verfahren nach Anspruch 8,
**dadurch gekennzeichnet,**
**daß** der Wert des Meßfehlers durch die auf dem Datenträger gespeicherten Informationen berechnet werden kann.

**10.** Verfahren nach einem der Ansprüche 8 oder 9,
**dadurch gekennzeichnet,**
**daß** der korrigierte Wert der gestörten Komponente auf dem Ausdruck und/oder auf dem Display der elektronischen Datenverarbeitung erscheint.

**11.** Verfahren nach Anspruch 8 bis 10,
**dadurch gekennzeichnet,**
**daß** der korrigierte Wert mit einem Toleranzbereich angegeben wird.

**12.** Verfahren nach einem der Ansprüche 10 oder 11,
**dadurch gekennzeichnet,**
**daß** die Korrektur des Meßwertes nur innerhalb bestimmter Toleranzgrenzen automatisch erfolgt.

**13.** Verfahren nach einem der vorhergehenden Ansprüche, wobei die zu bestimmende Komponente ausgewählt wird aus (a) der Analytgruppe, die entsprechend dem biochemischen Reaktionstypus von GOT/GPT detektiert wird und/oder (b) zu einer Analytgruppe gehört, die aus Gesamtprotein, Albumin, LDH, Kalium, Gesamtcholesterin, freiem Cholesterin, Harnsäure, Triglyceriden, Natrium, Chlorid, β-Lipoproteinen, Thymoltrübungstesten, Zinksulfattrübungstesten, Phospholipiden und freien Fettsäuren besteht.

**14.** Verfahren nach einem der vorhergehenden Ansprüche, wobei eine Komponente bestimmt wird, deren Konzentration in roten Blutzellen höher ist als im Blutserum oder im Blutplasma.

**Claims**

**1.** Method for the analysis of a medical sample while avoiding error contributions due to haemolysis in which, prior to the main reaction of a component present in the sample, a pre-reaction is produced and measured by which the degree of haemolysis in the sample is determined and the result of the sample to be determined that is subsequently obtained is corrected by this error contribution using the relationship (correlation) that has been found between the degree of haemolysis and the error contribution.

**2.** Method as claimed in claim 1,
**characterized in that**
the pre-reaction is also determined photometrically.

**3.** Method as claimed in claim 1 or 2,
**characterized in that**
the photometric determination is carried out bichromatically at 340/405 nm and the actual measured value results by forming the difference between the results of the two wavelengths.

**4.** Method as claimed in one of the previous claims,
**characterized in that**
the pre-reaction is started by addition of a reagent which contains NADH and LDH.

**5.** Method as claimed in claim 4,
**characterized in that**
the reagent additionally contains MDH.

**6.** Method as claimed in one of the previous claims,
**characterized in that**
the relationship between the degree of haemolysis determined by the pre-reaction and the contribution to the measurement error caused by the interfering components is ensured on a broad basis for a large group of test persons with a heterogeneous state of health, age and sex.

**7.** Method as claimed in one of the previous claims,
**characterized in that**
the correlation of the pre-reaction with the determination reaction with regard to the component to be determined is achieved by the formula

$$rate_{substance/sample} = rate_{total} - rate_{pre-reaction} - rate_{substance/erythrocytes}$$

in which substance denotes the component to be determined in the sample.

**8.** Method as claimed in one of the previous claims,
**characterized in that**
the formula necessary for calculating the corrected value is stored on a data carrier and this is used to automatically correct the analytical results with the aid of electronic data processing.

**9.** Method as claimed in claim 8,
**characterized in that**
the value of the measurement error can be calculated from the information stored on the data carrier.

**10.** Method as claimed in one of the claims 8 or 9,
**characterized in that**
the corrected value of the component that is interfered with appears on the print-out and/or on the display of the electronic data processing.

**11.** Method as claimed in claims 8 to 10,
**characterized in that**
the corrected value is stated with a tolerance range.

**12.** Method as claimed in one of the claims 10 or 11,
**characterized in that**
the correction of the measured value is only carried out automatically within particular tolerance limits.

**13.** Method as claimed in one of the previous claims, wherein the component to be determined is selected from (a) the analyte group which can be detected in a manner corresponding to that of the GOT/GPT biochemical reaction type and/or (b) which belongs to a group of analytes comprising total protein, albumin, LDH, potassium, total cholesterol, free cholesterol, uric acid, triglycerides, sodium, chloride, β-lipoproteins, thymol turbidity tests, zinc sulphate turbidity tests, phospholipids and free fatty acids.

14. Method as claimed in one of the previous claims,
wherein a component is determined the concentration of which is higher in red blood cells than in blood serum or in blood plasma.

**Revendications**

1. Procédé d'analyse d'un échantillon médical évitant des contributions perturbatrices dues à une hémolyse, dans lequel, avant la réaction principale, une réaction préliminaire est générée et mesurée pour un composant contenu dans l'échantillon, de manière à déterminer le degré d'hémolyse de l'échantillon et à corriger le résultat obtenu par la suite pour l'échantillon à doser par cette contribution perturbatrice en exploitant la relation trouvée (corrélation) entre le degré d'hémolyse et la contribution perturbatrice.

2. Procédé selon la revendication 1, **caractérisé en ce que** la réaction préliminaire est également réalisée de façon photométrique.

3. Procédé selon la revendication 1 ou 2, **caractérisé en ce que** le dosage photométrique a lieu à 340/405 nm de manière bichromatique et la valeur mesurée proprement dite est produite par la soustraction des résultats des deux longueurs d'onde.

4. Procédé selon l'une quelconque des revendications précédentes, **caractérisé en ce que** la préparation de la réaction préliminaire a lieu par addition d'un réactif contenant du NADH et de la LDH.

5. Procédé selon la revendication 4, **caractérisé en ce que** le réactif contient en outre de la MDH.

6. Procédé selon l'une quelconque des revendications précédentes, **caractérisé en ce que** la relation entre le degré d'hémolyse déterminé par la réaction préliminaire et la contribution d'erreur de mesure due aux composants perturbateurs est garanti largement pour un grand groupe de sujets d'expérimentation présentant des états de santé, des âges et des sexes hétérogènes.

7. Procédé selon l'une quelconque des revendications précédentes, **caractérisé en ce que** la corrélation entre la réaction préliminaire et la réaction de dosage concernant le composant à doser s'effectue par l'intermédiaire de la formule

$$\text{Rapport}_{\text{substance/échantillon}} = \text{Rapport}_{\text{total}} - \text{Rapport}_{\text{réaction\_préliminaire}} - \text{Rapport}_{\text{substance/érythrocytes}}$$

où substance désigne le composant à doser dans l'échantillon.

8. Procédé selon l'une quelconque des revendications précédentes, **caractérisé en ce que** la formule nécessaire au calcul de la valeur corrigée est stockée sur un support de données et celui-ci est utilisé pour une correction automatique des résultats d'analyse à l'aide du traitement électronique des données.

9. Procédé selon la revendication 8, **caractérisé en ce que** la valeur de l'erreur de mesure peut être calculée avec les informations stockées sur le support de données.

10. Procédé selon l'une quelconque des revendications 8 ou 9, **caractérisé en ce que** la valeur corrigée du composant perturbé apparaît sur la copie papier et/ou à l'affichage du traitement électronique des données.

11. Procédé selon les revendications 8 à 10, **caractérisé en ce que** la valeur corrigée est indiquée avec une plage de tolérance.

12. Procédé selon l'une quelconque des revendications 10 ou 11, **caractérisé en ce que** la correction de la valeur mesurée n'est effectuée automatiquement qu'à l'intérieur de certaines limites de tolérance.

**13.** Procédé selon l'une quelconque des revendications précédentes, dans lequel le composant à doser est choisi dans (a) le groupe de substances à analyser qui est détecté selon le type de réaction biochimique GOT/GPT, et/ ou (b) appartient à un groupe de substances à analyser qui se compose de protéines totales, d'albumine, de LDH, de potassium, de cholestérol total, de cholestérol libre, d'acide urique, de triglycérides, de sodium, de chlorure, de bêta-lipoprotéines, d'analyses néphélométriques de thymol, d'analyses néphélométriques de sulfate de zinc, de phospholipides et d'acides gras libres.

**14.** Procédé selon l'une quelconque des revendications précédentes, dans lequel un composant est dosé, dont la concentration en globules rouges est supérieure à celle dans le sérum sanguin ou dans le plasma sanguin.

# Fig.1

GOT - Entstörung
WIEDERFINDUNG IN ABHÄNGIGKEIT VON PROBENAKTIVITÄT
UND STÖRUNG

QUADRATE —> PROBENAKTIVITÄT OHNE ENTSTÖRUNG
STERNE ——> PROBENAKTIVITÄT MIT ENTSTÖRUNG

Figur 2

ΔE = 340 - 405

R2-Zugabe

Probe + R1

Rate$_{Vorreaktion}$

Rate$_{tot}$

t

$$Rate_{tot} = Rate_{GOT/Serum} + Rate_{GOT/Erythrozyten} + Rate_{Vorreaktion}$$

EP 0 695 805 B1

## Aufstockungsversuche bei 20 Seren mit einem Hämolysat ohne Entstörung

| Seren | Serum(U/l) | WDF % | +50 * | +100 * | +150 * | +200 * | +250 * | +300 * | +400 * | +500 * |
|---|---|---|---|---|---|---|---|---|---|---|
| 1 | 24,15 | 100,0% | 101,6% | 107,7% | 108,9% | 116,3% | 124,5% | 130,3% | 141,7% | 152,5% |
| 2 | 20,37 | 100,0% | 105,2% | 109,8% | 114,1% | 122,6% | 126,7% | 136,0% | 150,3% | 166,0% |
| 3 | 16,08 | 100,0% | 103,7% | 110,9% | 117,4% | 122,6% | 133,5% | 141,4% | 165,3% | 177,9% |
| 4 | 16,96 | 100,0% | 104,6% | 112,6% | 119,2% | 128,9% | 133,8% | 143,0% | 169,3% | 182,0% |
| 5 | 21,66 | 100,0% | 102,7% | 110,6% | 112,0% | 118,8% | 125,2% | 130,4% | 148,6% | 157,7% |
| 6 | 17,18 | 100,0% | 102,0% | 110,9% | 118,1% | 128,5% | 132,0% | 146,6% | 160,0% | 174,4% |
| 7 | 21,21 | 100,0% | 103,7% | 107,4% | 113,5% | 117,9% | 125,5% | 130,6% | 145,0% | 158,6% |
| 8 | 33,15 | 100,0% | 106,7% | 101,9% | 110,3% | 112,5% | 115,4% | 120,9% | 132,5% | 139,2% |
| 9 | 48,05 | 100,0% | 103,0% | 104,6% | 107,6% | 109,9% | 113,1% | 115,3% | 123,0% | 127,2% |
| 10 | 24,16 | 100,0% | 104,2% | 107,2% | 115,6% | 119,1% | 127,2% | 130,1% | 143,8% | 154,0% |
| 11 | 34,48 | 100,0% | 104,4% | 106,0% | 108,9% | 111,5% | 117,0% | 119,6% | 131,8% | 134,4% |
| 12 | 33,75 | 100,0% | 106,0% | 106,0% | 110,5% | 114,0% | 117,5% | 121,7% | 134,4% | 140,4% |
| 13 | 43,17 | 100,0% | 102,1% | 105,5% | 109,2% | 109,9% | 113,2% | 115,5% | 122,5% | 125,9% |
| 14 | 85,86 | 100,0% | 103,7% | 104,7% | 106,5% | 106,9% | 110,3% | 111,8% | 115,2% | 118,3% |
| 15 | 221,20 | 100,0% | 99,9% | 100,8% | 102,6% | 102,1% | 103,6% | 105,1% | 102,4% | 105,2% |
| 16 | 59,00 | 100,0% | 105,3% | 103,7% | 105,7% | 107,5% | 109,3% | 112,2% | 117,3% | 122,3% |
| 17 | 57,42 | 100,0% | 101,4% | 106,5% | 105,6% | 106,7% | 110,2% | 111,3% | 117,7% | 122,5% |
| 18 | 20,32 | 100,0% | 106,6% | 110,9% | 117,2% | 123,3% | 130,3% | 139,9% | 155,5% | 170,5% |
| 19 | 14,54 | 100,0% | 105,9% | 112,8% | 121,0% | 129,3% | 144,1% | 147,3% | - | - |
| 20 | 13,72 | 100,0% | 108,1% | 113,6% | 124,1% | 131,9% | 144,8% | - | - | - |

* mg/dl Hämoglobin

Figur 4

## got

## Rank 5  Eqn 1003  $y=a+bx+cx^2$

Figur 5

Vergleich: BM entstört (Rate-plus) gegen BM- Sollwerte

E / t  —  DIAGRAMM                                          Fig.6

GOT Hb - KONZENTRATIONSREIHE WL 340 nm

ZEIT ( MINUTEN )

EXTINKTION ( - )

------------------- 179430 0002 22 0003 Cal
— — — — — — —  199430 0002 22 0005 SERUM
..................... 179430 0002 22 0006 SERUM + 50 Hb
—·—·—·—·—·—  179430 0002 22 0007 SERUM + 100 Hb

— — — — — — 179430 0002 22 0008 SERUM + 150 Hb
– – – – – – 179430 0002 22 0009 SERUM + 200 Hb
—··—··—··— 179430 0002 22 0010 SERUM + 250 Hb
——————— 179430 0002 22 0011 SERUM + 300 Hb

EP 0 695 805 B1

Fig.7

# E / t — DIAGRAMM

## GOT Hb - KONZENTRATIONSREIHE WL 405 nm

Legend:

— — — — — — — — — — 179430 0002 23 0003 Cal
———————————— 179430 0002 23 0005 SERUM
················································ 179430 0002 23 0006 SERUM + 50 Hb
—·—·—·—·—·—·— 179430 0002 23 0007 SERUM + 100 Hb

— — — — — 179430 0002 23 0008 SERUM + 150 Hb
— — — — — — — 179430 0002 23 0009 SERUM + 200 Hb
—··—··—··—·· 179430 0002 23 0010 SERUM + 250 Hb
———————— 179430 0002 23 0011 SERUM + 300 Hb

Axis labels: EXTINKTION (-) ; ZEIT ( MINUTEN )

EP 0 695 805 B1